# EUROPEAN PATENT APPLICATION

(11) **EP 4 834 265 A1**
(43) Date of publication of application: **04.11.2026**
(21) Application number: 25173901.7
(22) Date of filing: 02.05.2025
(51) Int. Cl.: A61B 18/24, A61B 8/08, A61B 17/221, A61B 18/14

(54) **SYSTEM AND METHOD FOR REMOVING MATERIAL FROM BODILY LUMEN USING CAPTURE TOOL**

(71) Applicant: Koninklijke Philips N.V., 5656 AE Eindhoven (NL)
(72) Inventor: MIEZWA, Megan Ann, Eindhoven (NL); VAN DER HORST, Arjen, Eindhoven (NL); KURANE, Aditee, Eindhoven (NL); PERRIN, Chad, Eindhoven (NL); MACCONNELL, Michael Bryan, Eindhoven (NL)
(74) Representative: Philips Intellectual Property & Standards

(57) **Abstract**

A catheter system is provided for removing material from a bodily lumen of a subject. The catheter system includes an outer catheter configured for insertion in the bodily lumen, where the outer catheter defines a first lumen and includes a cutting tool at a distal tip of the outer catheter; and an inner catheter configured for insertion in the bodily lumen through the first lumen of the outer catheter, where the inner catheter comprises a capture tool configured to extend from the distal tip of the outer catheter and to capture the material. The outer catheter is further configured to advance over the inner catheter toward the captured material and to remove the captured material from the bodily lumen while the material is captured by the capture tool.

## Description

### FIELD OF THE INVENTION

The invention relates to the field of medical treatment for intravascular lesions, and more specifically to removing lesions in a vessel of a subject using catheters.

### BACKGROUND

Venous thromboembolism, which includes deep vein thrombosis (DVT), is a major contributor to the global disease burden and is the third most common cardiovascular pathology after coronary artery disease and stroke. Lower extremity DVT (LEDVT) can block the venous lumen and leads to venous congestion, swelling, and lower extremity venous valve function damage, resulting in post-thrombotic syndrome (PTS).

Standard treatments of venous obstructions may include the use of balloons, stents, lytics, and mechanical aspiration. Stents restore flow in PTS cases, but treatment location is limited to the iliac veins and a permanent implant is left behind. The implanted stents, and particularly those used to treat DVTs with concurrent inflow disease, are at risk of re-occluding. Those that do occlude have very limited re-interventional options aside from surgical removal of the stent and may significantly impair the patient's quality of life.

Mechanical thrombectomy treatments involve collection and extraction of the clot, either by mechanical entanglement and transport, or through use of vacuum aspiration. Currently, effective ways of removing clots that have progressed in chronicity to create symptoms of PTS using endovascular methods are lacking. Such post-thrombotic lesions are very tough and wall-adherent, and therefore difficult to remove. When post-thrombotic scarring is located below the common femoral vein, treatment options are limited to percutaneous transluminal angioplasty (PTA), and treatment of these vessels is usually a last attempt to delay the onset of stent occlusion and ultimately re-intervention.

High-pressure balloon venoplasty is currently the only method used to increase lumen area of a vessel by pushing the disease to the side and breaking collagen webs inside the vessel. However, high-pressure balloon venoplasty is not very effective, and the high pressure applied within the lumen may result in vessel damage and re-thrombosis.

### SUMMARY

According to a representative embodiment, a catheter system is provided for removing material from a bodily lumen of a subject. The catheter system includes an outer catheter configured for insertion in the bodily lumen, where the outer catheter defines a first lumen and includes a cutting tool at a distal tip of the outer catheter; and an inner catheter configured for insertion in the bodily lumen through the first lumen of the outer catheter, where the inner catheter comprises a capture tool configured to extend from the distal tip of the outer catheter and to capture the material. The outer catheter is further configured to advance over the inner catheter toward the captured material and to remove the captured material from the bodily lumen while the material is captured by the capture tool.

According to another representative embodiment, a method is provided for removing material in a bodily lumen of a subject using a catheter system, including an outer catheter defining a first lumen and including a cutting tool at a distal tip of the outer catheter, and an inner catheter including a capture tool. The method includes advancing the outer catheter through the bodily lumen to the material; advancing the inner catheter through the first lumen of the outer catheter to the material; extending the capture tool of the inner catheter from the distal tip of the outer catheter; capturing the material by operation of the capture tool; removing the captured material from the bodily lumen using the cutting tool at the distal tip of the outer catheter; and retracting the inner catheter through the first lumen of the outer catheter to retrieve the captured material from the bodily lumen while the material is captured by the capture tool.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like tools.
FIG. 1 is a simplified block diagram and plan view of a system for removing material from a bodily lumen of a subject, according to a representative embodiment.
FIG. 2 is a simplified plan view of a catheter system including a laser cutting tool and a gripper as the capture tool for removing lesion material from a vessel of a subject, according to a representative embodiment.
FIG. 3 is a simplified plan view of a catheter system including a laser source and a snare as the capture tool for removing lesion material from a vessel of a subject, according to a representative embodiment.
FIG. 4 is a simplified plan view of a catheter system including a laser source and a helix as the capture tool for removing lesion material from a vessel of a subject, according to a representative embodiment.
FIG. 5 is a simplified plan view of a catheter system including a laser source and an expandable mesh as the capture tool for removing lesion material from a vessel of a subject, according to a representative embodiment.
FIG. 6 is a simplified plan view of a catheter system including a laser source, a gripper, and an IVUS imaging device on an inner catheter, according to a representative embodiment.
FIG. 7 is a simplified plan view of a catheter system including a laser source, a gripper, and an IVUS imaging device on an outer catheter, according to a representative embodiment.
FIG. 8 is a flow diagram of a method of removing material from a bodily lumen in a subject, according to a representative embodiment.
FIGs. 9A to 9I are simplified plan views of the catheter system removing lesion material from a vessel of a subject corresponding to the steps in the flow diagram of FIG. 8, respectively, according to a representative embodiment.
FIG. 10 is a simplified plan view of a catheter system including a radio frequency (RF) cutting tool and a gripper as the capture tool for removing lesion material from a vessel of a subject, according to a representative embodiment.
FIG. 11 is a simplified plan view of a catheter system including a high-intensity focused ultrasound (HIFU) cutting tool and a gripper as the capture tool for removing lesion material from a vessel of a subject, according to a representative embodiment.
FIG. 12 is a simplified plan view of a catheter system including a mechanical cutting tool and a gripper as the capture tool for removing lesion material from a vessel of a subject, according to a representative embodiment.
FIG. 13 is a simplified plan view of a catheter system including a laser cutting tool and a gripper as the capture tool, and pull wires for manipulating the gripper, according to a representative embodiment.

### DETAILED DESCRIPTION

In the following detailed description, for the purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms "a," "an" and "the" are intended to include both singular and plural forms, unless the context clearly dictates otherwise. Additionally, the terms "comprises," "comprising," and/or similar terms specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as not to obscure the description of the example embodiments. Such methods and apparatuses are within the scope of the present disclosure.

Generally, the various embodiments described herein provide a system and method for capturing material, such as a lesion and collagen webs, a focal luminal obstruction (such as a flap) or a septated luminal obstruction, within a bodily lumen in a safe effective manner using a first catheter with a cutting tool and a second catheter with a capture tool. The capture tool is configured to grip the material to be removed, which also aligns the cutting tool for ablation and removal of the material. The catheter and cutting tool are used in an interventional setting, such as a catheterization or vascular laboratory, may be guided via venography (x-ray imaging) or ultrasound, for example.

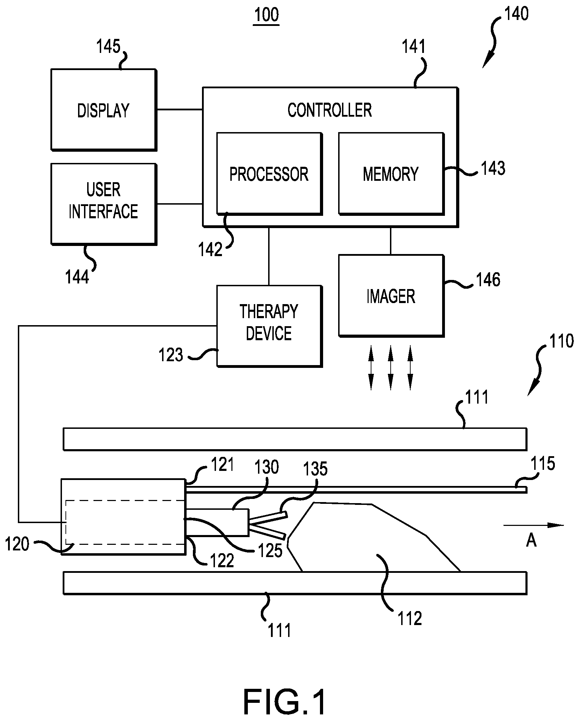

FIG. 1 is a simplified block diagram and plan view of a system for removing material from a bodily lumen of a subject, according to a representative embodiment.

Referring to FIG. 1, the system 100 includes a catheter system 110 configured to be inserted in a bodily lumen 111 of the subject (patient) and an optional control system 140 configured optionally to control all or part of the operation to capture and remove (debulk) material 112 from the bodily lumen 111. For purposes of illustration, the material 112 is shown within the bodily lumen 111, where the material 112 at least partially occludes the subject's blood flow through the bodily lumen 111. The catheter system 110 is shown positioned within the bodily lumen 111 adjacent to the material 112 during treatment for removing the material 112, as discussed below.

In various embodiments, the bodily lumen 111 may be any lumen of the subject through which a minimally invasive procedure may be performed using the catheter system 110, and the material 112 may be any object or tissue removable from the bodily lumen 111 by the catheter system 110. For example, the bodily lumen 111 may be a vessel and the material 112 may be lesion material that is part of a lesion, which may be a mass within the vessel, such as a blood clot, thrombus, collagenous lesions/synechia or in-stent thrombosis or post-thrombotic lesion, or atherosclerotic plaque, fibrotic and calcified lesions, or total chronic total occlusion, discussed below. As another example, the bodily lumen 111 may be any lumen through which laparoscopic surgery may be performed, such as intestines, stomach or throat, for example, and the material 112 may be lesion or tumor material. As another example, the bodily lumen 111 may be an artery or chamber of the heart, for example, and the material 112 may be a structure of the heart to be removed and replaced, such as a valve. Of course, other types of the bodily lumen 111 and the material 112 may be involved without departing from the scope of the present teachings.

The catheter system 110 includes an outer catheter 120 (removal catheter) and an inner catheter 130 (capture catheter) configured for insertion in the bodily lumen 111, both of which are advanced in direction A through the bodily lumen 111 toward the material 112. In the depicted embodiment, the catheter system 110 further includes an optional guide wire 115 configured for initial insertion in the bodily lumen 111. The guide wire 115 may be advanced manually by a user (e.g., physician, clinical technician) of the catheter system 110, or automatically under control of a controller (e.g., controller 141, discussed below), as would be apparent to one skilled in the art. The outer catheter 120 is inserted in the bodily lumen 111 following the guide wire 115, and the inner catheter 130 is inserted in the bodily lumen 111 through the outer catheter 120, thereby also following the guide wire 115. In an embodiment, the guide wire 115 passes through a guide wire lumen 121 formed longitudinally in the outer catheter 120, and the inner catheter 130 passes through a catheter lumen 122 formed longitudinally in the outer catheter 120. The inner catheter 130 is moveable (slidable) within the catheter lumen 122 relative to the outer catheter 120, such that the inner catheter 130 and the outer catheter 120 may be advanced through the bodily lumen 111 independently of one another. In an embodiment, the inner catheter 130 may include two or more telescoping portions (not shown), which are separately moveable within the catheter lumen 122. The telescoping portions are formed such that the respective diameters of the telescoping portions are smaller in a distal direction toward the distal end of the inner catheter 130. In alternative configurations, the guide wire 115 may pass through the same lumen as the inner catheter 130 without departing from the scope of the present teachings.

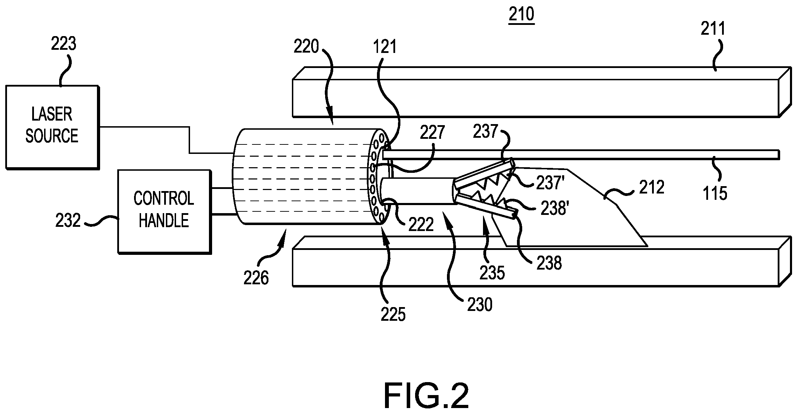

The inner catheter 130 includes a capture tool 135 at its distal end. The capture tool 135 is configured to physically grasp or otherwise secure the material 112, thereby immobilizing it. In the depicted embodiment, the capture tool 135 includes a gripper with a pair of opposable jaws operable to clamp onto the material 112 for capturing the material 112, as discussed below with reference to FIG. 2. Various other types of capture tool 135 may be incorporated without departing from the scope of the present teachings. The capture tool 135 is fixed to the distal end of the inner catheter 130, such that it is maneuverable by steering and manipulation of the inner catheter 130.

In an embodiment, at least one pull wire may be attached to a distal end of the inner catheter 130. The at least one pull wire is operable by the user to steer of the inner catheter 130 within the bodily lumen 111 to control placement of the capture tool 135 adjacent the material 112 for capturing the same. When capture tool 135 includes a gripper, as shown, the at least one pull wire (not shown) is attached immediately proximal to the capture tool 135. In another embodiment, at least one pull wire may be attached to a distal end of the outer catheter 120. The at least one pull wire (not shown) is likewise operable by the user to steer the outer catheter 120 within the bodily lumen 111, enabling steering of the outer catheter 120 (and thus the inner catheter 130 within the outer catheter 120) to the material 112 to control placement of the capture tool 135 adjacent the material 112 for capturing the same.

After the capture tool 135 captures the material 112, thereby immobilizing it, the outer catheter 120 is advanced over the inner catheter 130 toward the captured material 112, e.g., to remove the captured material 112 from the bodily lumen 111 while the material 112 is held in place by the capture tool 135. For example, the outer catheter 120 may remove at least a portion of the material 112 from a wall of the bodily lumen 111 by ablation or mechanical cutting while the material 112 is held by the capture tool 135. The removal of the material 112 is thus more efficient and results in less debris.

The outer catheter 120 includes a cutting tool 125 operable by a treatment device 123, where the cutting tool 125 and the treatment device 123 are configured to remove at least a portion of the material 112 that has been captured by the inner catheter 130, as discussed above. Examples of different types of cutting tools 125 include a laser cutting tool, a high-intensity focused ultrasound (HIFU) cutting tool, an electromagnetic (EM) wave cutting tool (e.g., radio frequency (RF) or microwave frequency (MW)), and a mechanical cutting tool, which are discussed in more detail below.

Generally, for laser treatment, the treatment device 123 includes a laser source configured to generate laser light, and the cutting tool 125 includes at least one optical fiber passing through the outer catheter 120 for emitting the laser light from a distal end of the outer catheter 120 to remove and/or ablate at least a portion of the material 112. For HIFU treatment, the treatment device 123 includes an ultrasound signal source configured to generate HIFU signals, and the cutting tool 125 includes at least one ultrasound transducer configured to emit HIFU signals from the distal tip of the outer catheter 120 to remove and/or ablate at least a portion of the material 112. For RF treatment, the treatment device 123 includes an RF signal source configured to generate RF electric current, and the cutting tool 125 includes at least one electrode or pair of electrodes connected to signal lines and configured to emit RF signals from the distal tip of the outer catheter 120 for removing and/or ablating the material 112. For mechanical treatment, the treatment device 123 includes a motor, and the cutting tool 125 includes a drive train passing through the mechanical catheter and a rotating cutter connected to the drive train at the distal end of the outer catheter 120 for mechanically debulking (cutting) the material 112. For all types of treatment, the treatment device 123 and the corresponding cutting tool 125 work in conjunction with the capture tool 135 to remove a least a portion of the material 112 from the bodily lumen 111 and/or a wall of the bodily lumen 111 and/or to destroy at least a portion of the material 112.

As mentioned above, the system 100 may further include the control system 140 for implementing and/or managing all or part of the processes described herein with regard to removing the material 112 using the catheter system 110. The control system 140 includes a controller 141 configured to interface with the user, control navigation and deployment of the catheter system 110 through the bodily lumen 111, and/or control imaging of the material 112 and the catheter system 110 by an imager 146. In the depicted embodiment, the controller 141 includes one or more processors indicated by processor 142 and one or more memories indicated by memory 143. The control system 140 may further include a user interface 144, a display 145, the imager 146, and the treatment device 123, discussed above. The imager 146 may be any compatible vascular imaging modality capable of showing the catheter system 110 and the material 112 within the bodily lumen 111 in real time during the treatment procedure. For example, the imager 146 may be an ultrasound imaging device, an x-ray imaging device, a computed tomography (CT) scanner, or a magnetic resonance (MR) imaging device. When the imager 146 is an ultrasound imaging device, it may include an ultrasound transducer probe that may be manipulated manually by the user, automatically by a robot under control of a robot controller (not shown), or a combination of both. The images may be displayed on the display 145.

The processor 142 communicates with the treatment device 123 through a treatment device interface (not shown) and with the imager 146 through an imaging interface (not shown), as would be apparent to one skilled in the art. The processor 142 is representative of one or more processing devices, and may be implemented by a general purpose computer, a central processing unit (CPU), a digital signal processor (DSP), a graphical processing unit, a computer processor, a microprocessor, a state machine, programmable logic device, field programmable gate arrays (FPGAs), application specific integrated circuits (ASICs), or combinations thereof, using any combination of hardware, software, firmware, hard-wired logic circuits, or combinations thereof. Any processor or processing unit herein may include multiple processors, parallel processors, or both. Multiple processors may be included in, or coupled to, a single device or multiple devices. The processor 142 may also refer to a collection of processors within a single computer system or distributed among multiple computer systems, such as in a cloud-based or other multi-site application. Programs have software instructions performed by one or multiple processors that may be within the same computing device or which may be distributed across multiple computing devices.

The memory 143 stores instructions executable by the processor 142 that, when executed, cause the processor 142 to implement all or part of various more processes, as discussed below. The memory 143 may include main memory and/or static memory, where such memories may communicate with each other and the processor 142 via one or more buses. The memory 143 may be implemented by any number, type and combination of random access memory (RAM) and read-only memory (ROM), for example, and may store various types of information, such as software algorithms, artificial intelligence (AI) machine learning models, and computer programs, all of which are executable by the processor 142. The various types of ROM and RAM may include any number, type and combination of computer readable storage media, such as a disk drive, flash memory, an electrically programmable read-only memory (EPROM), an electrically erasable and programmable read only memory (EEPROM), registers, a hard disk, a removable disk, tape, compact disk read only memory (CD-ROM), digital versatile disk (DVD), floppy disk, Blu-ray disk, a universal serial bus (USB) drive, or any other form of storage medium. The memory 143 is a tangible storage medium for storing data and executable software instructions, and is non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The memory 143 may store software instructions and/or computer readable code that enable performance of various functions. The memory 143 may be secure and/or encrypted, or unsecure and/or unencrypted.

The user interface 144 is configured to provide information and data output by the processor 142, the memory 143, the imager 146 and/or the treatment device 123 to the user and/or for receiving information and data input by the user. That is, the user interface 144 enables the user to enter data and to control or manipulate aspects of the processes described herein, and also enables the processor 142 to indicate the effects of the user's input, which may include control or manipulation of the catheter system 110, the treatment device 123, and/or the imager 146. All or a portion of the user interface 144 may be implemented by a graphical user interface (GUI). The user interface 144 may include one or more interface devices, such as a mouse, a keyboard, a trackball, a joystick, a microphone, a video camera, a touchpad, a touchscreen, voice or gesture recognition captured by a microphone or video camera, for example.

The display 145 may be a monitor such as a computer monitor, a television, a liquid crystal display (LCD), an organic light emitting diode (OLED), a flat panel display, a solid-state display, or a cathode ray tube (CRT) display, or an electronic whiteboard, for example. The display 145 includes a screen for viewing images of the catheter system 110 and the material 112 within the bodily lumen 111 of the subject during the procedure.

As mentioned above, the catheter system 110 may include different types of treatment devices and corresponding cutting tools. FIG. 2 is a simplified plan view of a catheter system including a laser cutting tool and a gripper as the capture tool for removing lesion material from a vessel of a subject, according to a representative embodiment.

Referring to FIG. 2, catheter system 210 is a laser catheter system shown positioned within a vessel 211 adjacent to lesion material 212 during treatment for removing the lesion material 212. As compared to FIG. 1, the vessel 211 is an example of the bodily lumen 111 and the lesion material 212 is an example of the material 112 to be removed from the bodily lumen 111. For purposes of illustration, the lesion material 212 is shown within the vessel 211, where the lesion material 212 at least partially occludes the subject's blood flow through the vessel 211. The lesion material 212 is part of a lesion, which may be a mass within the vessel 211, such as a blood clot, thrombus, collagenous lesions/synechia or in-stent thrombosis or post-thrombotic lesion, or atherosclerotic plaque, fibrotic and calcified lesions, or total chronic total occlusion, for example, although other types of lesions may be treated using the catheter system 210 without departing from the scope of the present teachings.

The catheter system 210 includes an outer (laser) catheter 220 and an inner catheter 230 configured for insertion through the vessel 211, e.g., along the optional guide wire 115. The inner catheter 230 passes through a catheter lumen 222 formed longitudinally in the outer catheter 220, and is moveable therein, as discussed above. In the depicted embodiment, the inner catheter 230 includes a gripper 235 as the capture tool 135.

For the catheter system 210, the cutting tool is a laser cutting tool 225 in the outer catheter 220, which delivers laser light generated by a laser source 223. An advantage of laser ablation, in particular, is that no particles are generated and no embolic protection devices are needed to prevent pulmonary embolisms. The laser cutting tool 225 includes one or more end-emitting optical fibers 226 (indicated by representative dashed lines) passing longitudinally through the outer catheter 220 and corresponding fiber openings 227 at the distal end of the outer catheter 220 for emitting the laser light generated by the laser source 223. The laser source 223 may be a gas laser (e.g. an excimer laser), a solid-state laser, or a semiconductor laser. The laser source 223 is capable of ultraviolet (UV) light generation, for example, having a wavelength in a range of about 126nm to about 360nm, or more specifically in a range of about 300nm to about 360nm. A 308nm excimer laser or 355nm solid state laser, in particular, may be used, both of which provide ablation without significantly heating the tissue (e.g., lesion material 212), which is broken down at the atomic level. Also, the 308nm excimer and 355nm solid state lasers do not generate large particles, which can be harmful since such particles may result in an embolism (e.g., in the pulmonary circulation). Alternatively, the laser source 223 may be configured to provide infrared light, which vaporizes water, such as a CO₂ laser, for example.

The gripper 235 is positioned at a distal end of the inner catheter 230. The gripper 235 includes a pair of opposable jaws 237 and 238 with corresponding teeth 237' and 238', respectively. The opposable jaws 237 and 238 are operable to clamp onto the lesion material 212 for physically capturing the lesion material 212 between the opposable jaws 237 and 238, thereby immobilizing it. In the depicted embodiment, the gripper 235 is fixed to the distal end of the inner catheter 230, although in alternative embodiments, the gripper 235 may be movable (slidable) within a lumen of the inner catheter 230. The catheter system 210 also includes a control handle 232 configured to control the gripper 235 on the inner catheter 230 through operation by the user, including opening and closing the pair of opposable jaws 237 and 238. For example, the gripper 235 may be configured to enable operation by the user placing their middle finger into a stationary loop for leverage and their index finger into a finger hole that can be pulled back to actuate a pull wire for deflecting the distal tip of the gripper 235, thus providing steerability. Simultaneously, the thumb is inserted into a thumb loop and can be pulled backward to articulate the opposable jaws 237 and 238 of the gripper 235, allowing for precise gripping control.

The laser light generated by the laser source 223 and delivered by the laser cutting tool 225 ablates the lesion material 212 that is captured, and thus immobilized, by the gripper 235. Use of the gripper 235 thus directs the laser light to the lesion and not the native blood vessel wall. The ablation removes and/or destroys at least a portion of the lesion material, including separating it from the wall of the vessel 211. In order to perform the ablation of the lesion material 212, the inner catheter 230 is advanced through the outer catheter 220 to position the gripper 235 adjacent to the lesion material 212. The inner catheter 230 and thus the gripper 235 are extended from the distal end of the outer catheter 220. The user manipulates the control handle 232 to open the opposable jaws 237 and 238 of the gripper 235, and advances the inner catheter 230 so that the opened opposable jaws 237 and 238 engage with the lesion material 212. The user then manipulates the control handle 232 to close the opposable jaws 237 and 238 onto the lesion material 212.

At this point, the user advances the outer catheter 220 over the inner catheter 230 to bring the fiber openings 227 of the optical fibers 226 into close proximity (e.g., contact) with the captured lesion material 212. The laser source 223 is activated to ablate the grasped portion of the lesion material 212 using the laser light transmitted via the optical fibers 226, separating at least a portion of the lesion material 212 from the vessel wall and/or destroying at least a portion of the lesion material 212. Since the lesion material 212 is held in place relative to the distal end of the outer catheter 220, the laser light is automatically aimed directly into the lesion material 212, thereby improving efficiency of the ablation process.

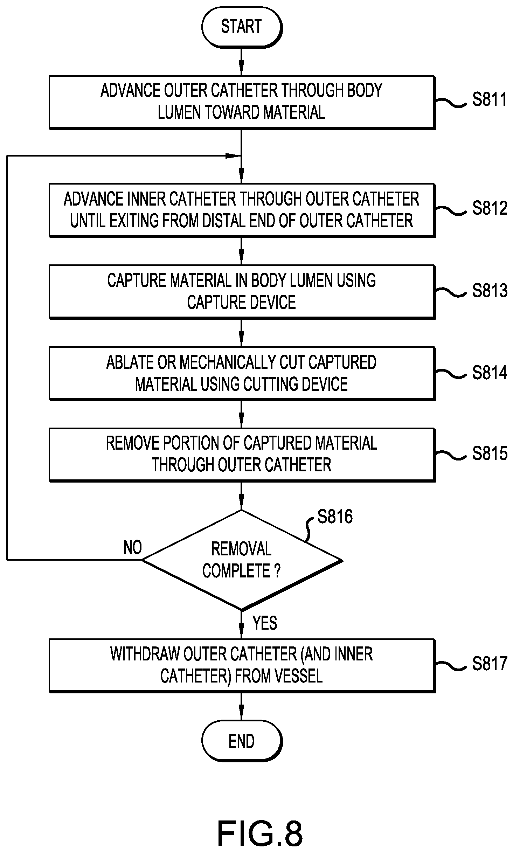

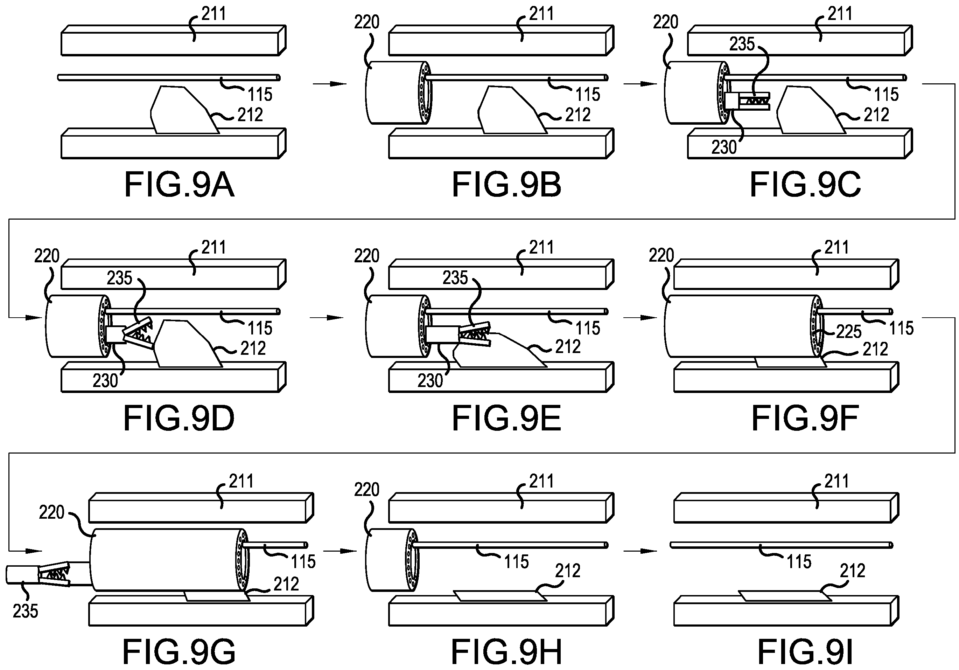

The process may be repeated as needed. Intravascular imaging may be incorporated to determine the level of completion of the ablation process after each round, as discussed below. For example, the lesion material 212 may be sufficiently eliminated when it has been reduced in size enough to enable substantially unrestricted blood flow. The process of removing the lesion material 212 is further discussed below with reference to FIGs. 8 and 9.

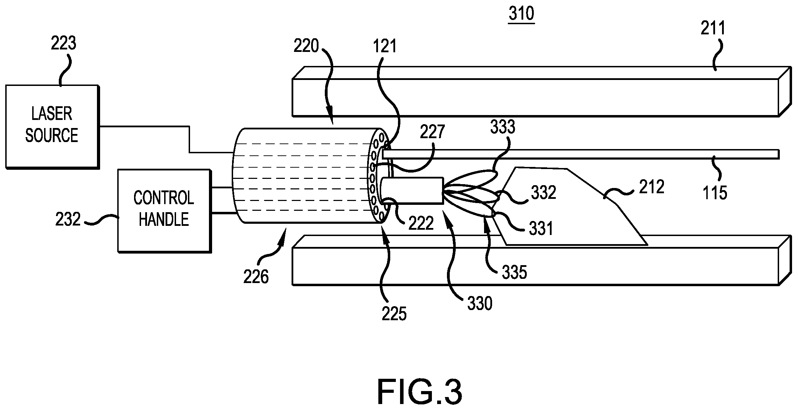

As mentioned above, different types of capture tool 135 may be positioned at the distal end of the inner catheter 230 for capturing the lesion material 212 without departing from the scope of the present teachings. For example, FIGs. 3, 4 and 5 are simplified plan views catheter systems including laser catheters and a snare, a helix and an expandable mesh as capture tools, respectively, according to representative embodiments.

Referring to FIG. 3, catheter system 310 is a laser catheter system shown positioned within the vessel 211 adjacent to lesion material 212 during treatment for removing the lesion material 212. The catheter system 310 includes the outer catheter 220 and an inner catheter 330 configured for insertion through the vessel 211, e.g., along the optional guide wire 115. The inner catheter 330 passes through the catheter lumen 222 formed longitudinally in the outer catheter 220, and is moveable therein, as discussed above.

In the depicted embodiment, the inner catheter 330 includes a snare 335 as the capture tool 135, which is positioned at a distal end of the inner catheter 330. The snare 335 includes one or more loops, indicated by representative first loop 331, second loop 332 and third loop 333. Of course, the snare 335 may include more or fewer loops without departing from the scope of the present teachings. The first to third loops 331 to 333 of the snare 335 are formed of a flexible material, such as nitinol or stainless steel, for example. In this way, the snare 335 is able to fold up inside the catheter lumen 222 of the outer catheter 220 when the outer catheter 220 is being maneuvered through the vessel 211, and to expand into a flower-like shape when the distal end of the inner catheter 330 is advanced through the catheter lumen 222 beyond the distal end of the outer catheter 220. Upon expansion, the snare 335 may be pressed against the lesion material 212 such that the first to third loops 331 to 333 flex and ensnare webbing and/or septation of the lesion material 212 in order to physically capture lesion material 212, thereby immobilizing it. In the depicted embodiment, the snare 335 is fixed to the distal end of the inner catheter 330, although in alternative embodiments, the snare 335 may be movable (slidable) within a lumen of the inner catheter 320.

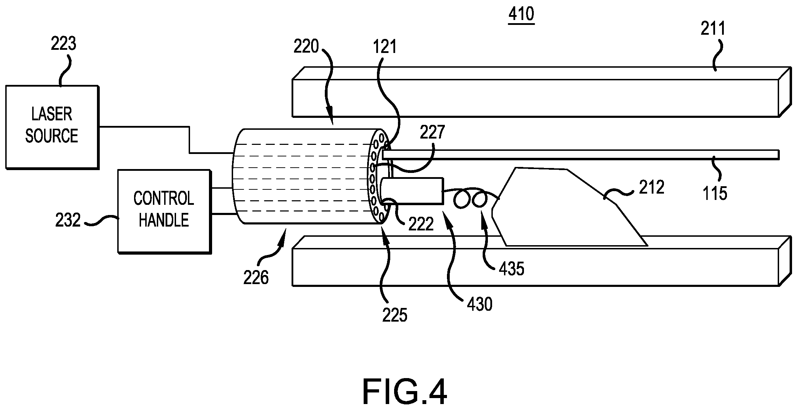

Referring to FIG. 4, catheter system 410 is a laser catheter system shown positioned within the vessel 211 adjacent to lesion material 212 during treatment for removing the lesion material 212. The catheter system 410 includes the outer catheter 220 and an inner catheter 430 configured for insertion through the vessel 211, e.g., along the optional guide wire 115. The inner catheter 430 passes through the catheter lumen 222 formed longitudinally in the outer catheter 220, and is moveable therein, as discussed above.

In the depicted embodiment, the inner catheter 430 includes a helix 435 as the capture tool 135, which is positioned at a distal end of the inner catheter 430. The helix 435 is corkscrew shaped, and includes a coiled wire with multiple turns ending in a pointed distal end. The helix 435 is formed of a substantially rigid material, such as nitinol, aluminum or stainless steel, for example. Also, the largest outer diameter of the helix 435 is smaller than an inner diameter of the catheter lumen 222. In this way, the helix 435 is able to fit inside the catheter lumen 222 of the outer catheter 220 when the outer catheter 220 is being maneuvered through the vessel 211. The helix 435 exits the distal end of the outer catheter 220 when the inner catheter 430 is advanced through the catheter lumen 222 beyond the distal end of the outer catheter 220. The helix 435 may be effectively screwed into the lesion material 212, e.g., by the user rotating the inner catheter 430, such that the helix 435 embeds within the lesion material 212, thereby immobilizing and anchoring into the lesion material 212. In the depicted embodiment, the helix 435 is fixed to the distal end of the inner catheter 430, although in alternative embodiments, the helix 435 may be movable (slidable) within a lumen of the inner catheter 430.

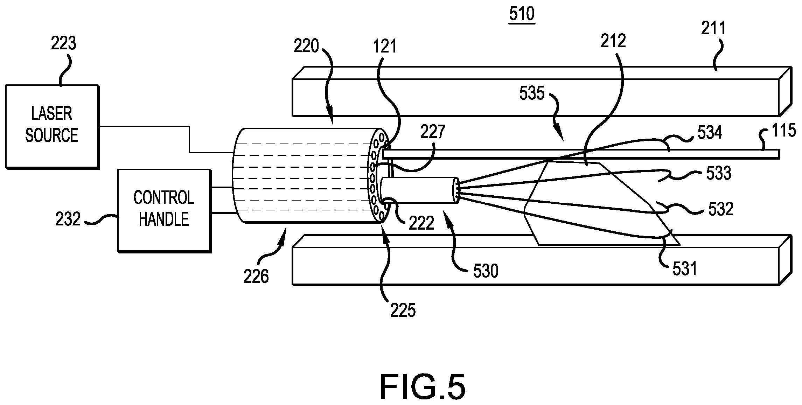

Referring to FIG. 5, catheter system 510 is a laser catheter system shown positioned within the vessel 211 adjacent to lesion material 212 during treatment for removing the lesion material 212. The catheter system 510 includes the outer catheter 220 and an inner catheter 530 configured for insertion through the vessel 211, e.g., along the optional guide wire 115. The inner catheter 530 passes through the catheter lumen 222 formed longitudinally in the outer catheter 220, and is moveable therein, as discussed above.

In the depicted embodiment, the inner catheter 530 includes an expandable mesh 535 as the capture tool 135, which is positioned at a distal end of the inner catheter 530. The expandable mesh 535 includes one or more anchors at one or more distal ends of the expandable mesh 535, indicated by representative first anchor 531, second anchor 532, third anchor 533 and fourth anchor 534, for example. The expandable mesh 535 is formed of a flexible material having shape memory properties, such as nitinol, for example. In this way, the expandable mesh 535 is able to fold up inside the catheter lumen 222 of the outer catheter 220 when the outer catheter 220 is being maneuvered through the vessel 211, and to expand outwardly into a pre-determined shape when the distal end of the inner catheter 530 is advanced through the catheter lumen 222 beyond the distal end of the outer catheter 220. When the expandable mesh 535 expands, the first to fourth anchors 531 to 534 anchor into respective portions of the lesion material 212 in order to capture the lesion material 212, thereby immobilizing it. In the depicted embodiment, the expandable mesh 535 is fixed to the distal end of the inner catheter 530, although in alternative embodiments, the expandable mesh 535 may be movable (slidable) within a lumen of the inner catheter 530.

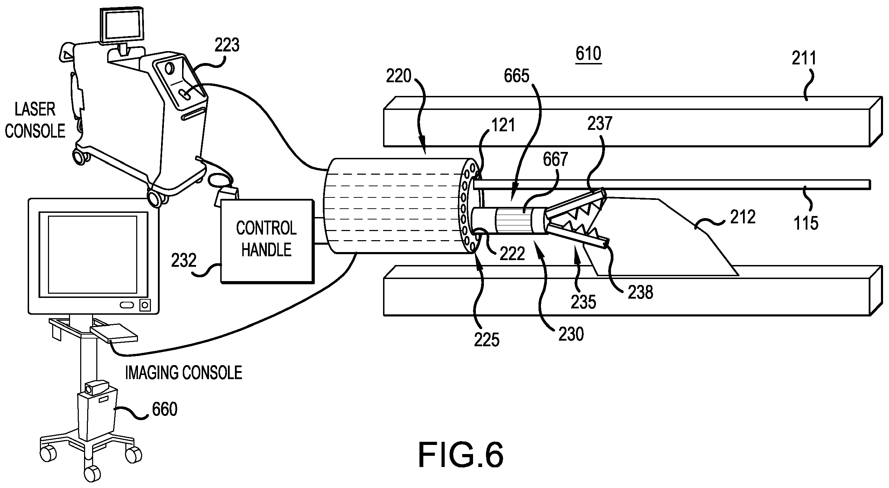

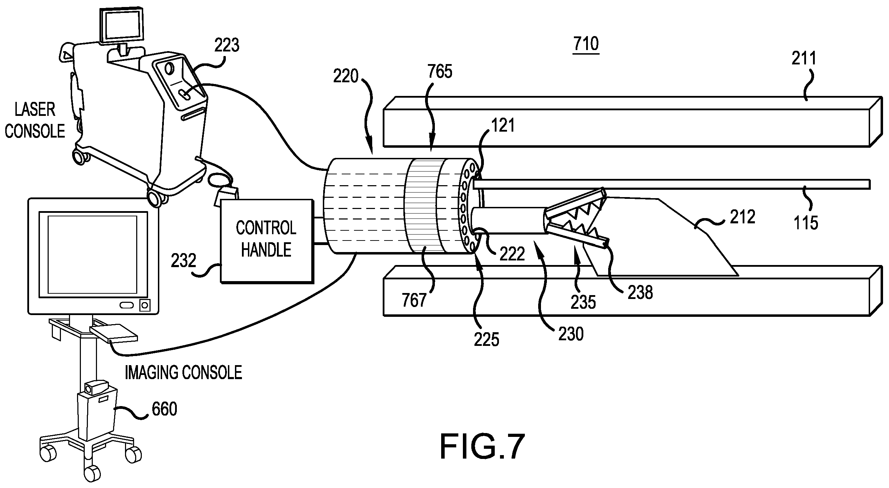

The removal process may be further enhanced by including intravascular imaging with the catheter system, such as IVUS imaging or optical imaging (e.g., optical coherence tomography (OCT) imaging), for example. FIG. 6 is a simplified plan view a catheter system including a laser source for cutting/ablating lesion material, a gripper for capturing the lesion material from a vessel, and an IVUS imaging device on an inner catheter for performing intervascular imaging, and FIG. 7 is a simplified plan view a catheter system including a laser source for cutting/ablating lesion material, a gripper for capturing the lesion material from a vessel, and an IVUS imaging device on an outer catheter for performing intervascular imaging, according to representative embodiments. Both catheter systems may include optical imaging devices in place of the IVUS imaging devices, without departing from the scope of the present teachings. An optical imaging device may include a camera (e.g., fiber optic camera) or a Michelson interferometer, for example, configured to receive reflected light via the optical fibers 226 to provide the intravascular optical images.

Referring to FIG. 6, catheter system 610 is the same as the catheter system 210 with the addition of an inner IVUS imager 665 configured to provide ultrasound images of the vessel 211 and the lesion material 212 throughout the removal procedure. That is, the catheter system 610 includes the outer catheter 220 and the inner catheter 230 configured for insertion in the vessel 211, e.g., following the guide wire 115. As discussed above, the outer catheter 220 includes the laser cutting tool 225 with one or more end-emitting optical fibers 226 passing longitudinally through the outer catheter 220 for emitting the laser light generated by the laser source 223 from the corresponding fiber openings 227 at the distal end of the outer catheter 220. The inner catheter 230 includes the gripper 235 at its distal end with the opposable jaws 237 and 238 operable to clamp onto the lesion material 212 for physically capturing and thereby immobilizing at least a portion of the lesion material 212.

In the depicted embodiment, the inner catheter 230 further includes the inner IVUS imager 665, mention above, positioned proximal to the gripper 235. The inner IVUS imager 665 includes a set of ultrasound transducers 667 arranged around an outer perimeter of the end portion of the inner catheter 230. The ultrasound transducers 667 receive electrical signals from ultrasound imaging system 660 via signal lines (not shown) traversing the inner catheter 230, convert the electrical signals to ultrasonic waves transmitted toward the vessel walls of the vessel 211 and/or the lesion material 212, and convert reflected ultrasonic waves back to electrical signals, which are sent to the ultrasound imaging system 660 via the signal lines. The placement of the inner IVUS imager 665 near the distal end of the inner catheter 230 enables imaging of the vessel walls and/or the lesion material 212 as the inner catheter 230 is advanced toward and contacts the lesion material 212. Also, after treatment, the inner catheter 230 may be advanced toward the original location of the lesion material 212, after having been withdrawn into the outer catheter 220, and the inner IVUS imager 665 may be used to assess the at least partially removed lesion material 212 for comparison it to the pre-treatment geometry.

Referring to FIG. 7, catheter system 710 is the same as the catheter system 610 except that, instead of the inner IVUS imager 665, an outer IVUS imager 765 is positioned on the outer catheter 220 proximate to the distal end of the outer catheter 220. The outer IVUS imager 765 is configured to provide ultrasound images of the vessel 211 and the lesion material 212 throughout the removal procedure.

In the depicted embodiment, the outer IVUS imager 765 includes a set of ultrasound transducers 767 arranged around an outer perimeter of the outer catheter 220. The ultrasound transducers 767 receive electrical signals from ultrasound imaging system 660 via signal lines (not shown) traversing the outer catheter 220, convert the electrical signals to ultrasonic waves transmitted toward the vessel walls of the vessel 211 and/or the lesion material 212, and convert reflected ultrasonic waves back to electrical signals, which are sent to the ultrasound imaging system 660 via the signal lines. The placement of the outer IVUS imager 765 near the distal end of the outer catheter 220 enables imaging of the vessel walls and/or the lesion material 212 as the outer catheter 220 is advanced toward and contacts the lesion material 212. This is particularly the case when the inner catheter 230 is drawn back into the outer catheter 220 with the captured portion of the lesion material in the gripper 235, bringing the capture portion of the lesion material into close proximity to the distal end of the outer catheter 220. Also, after treatment, the outer catheter 220 may be advanced toward the original location of the lesion material 212 and the outer IVUS imager 765 may be used to assess the at least partially removed lesion material 212 for comparison to the pre-treatment geometry.

Although shown in separate embodiments, it is understood that the inner IVUS imager 665 and the outer IVUS imager 765 may be included on a single catheter system in an alternative embodiment. Further, the inner IVUS imager 665 and/or the outer IVUS imager 765 may be included with any of the embodiments of the catheter systems discussed herein. In another embodiment, both an IVUS imager may be included in an additional catheter, other than the inner catheter 230 or the outer catheter 220, without departing from the scope of the present teachings.

FIG. 8 is a flow diagram of a method of removing material from a bodily lumen in a subject, according to a representative embodiment. FIGs. 9A to 9I are simplified plan views the catheter system removing material from a bodily lumen of a subject corresponding to the steps in the flow diagram of FIG. 8, respectively, according to a representative embodiment. For ease of illustration, the method is described with reference to the catheter system 210, discussed above. The outer catheter 220 includes the laser cutting tool 225 with one or more end-emitting optical fibers 226 for emitting the laser light generated by the laser source 223 from the corresponding fiber openings 227, and the inner catheter 230 includes the gripper 235 at its distal end with the opposable jaws 237 and 238 operable to clamp onto the lesion material 212 for physically capturing and thereby immobilizing the lesion material 212. It is understood, however, that the general steps depicted in FIG. 8 and FIGs. 9A to 9I may be implemented for other types of catheter systems with different cutting tools and/or capture tools discussed herein, as well as other types of bodily lumens, without departing from the scope of the present teachings.

Referring to FIG. 8, in block S811, the outer catheter is inserted in the vessel of the subject and advanced through the vessel toward the lesion material until it is near a proximal side of the lesion material. The advancement of the outer catheter may stop when its distal end is positioned a short distance from the lesion material 212 (e.g., a distance about 10-20mm) that enables maneuvering of the inner catheter, for example. In an embodiment, the outer catheter may be advanced along a guide wire previously inserted in the vessel. In this regard, FIG. 9A shows the guide wire 115 of the catheter system 210 that has been inserted in the vessel 211 past the lesion material 212. FIG. 9B shows the outer catheter 220 advancing along the guide wire 115 through the vessel 211 to near the proximal side of the lesion material 212.

In block S812, the inner catheter is advanced through the outer catheter toward the material until the capture tool exits the distal end of the outer catheter. All or a portion of the inner catheter may be positioned within the outer catheter while the outer catheter is being advanced through the vessel, or the inner catheter may be inserted into the outer catheter after the outer catheter has been placed near the lesion material. FIG. 9C shows the inner catheter 230 being advanced through the outer catheter 220 until the gripper 235 of the inner catheter 230 exits the distal end of the outer catheter 220 a short distance from the lesion material 212.

In block S813, the material is captured using the capture tool operated via the inner catheter and a control handle. That is, the capture tool is advanced into the material using the inner catheter and operated to clamp onto, imbed in or otherwise secure the material. FIG. 9D shows the opposable jaws 237 and 238 of the gripper 235 opening as the inner catheter 230 is advanced toward the lesion material 212, and FIG. 9E shows the opposable jaws 237 and 238 of the gripper 235 closing onto the lesion material 212 in order to capture it.

In block S814, the captured material is removed by ablation or mechanical cutting using the cutting tool operated via the outer catheter, separating at least a portion of the material from the wall of the bodily lumen and/or destroying at least a portion of the material. For example, the outer catheter may be advanced over the inner catheter while the cutting tool is operated to ablate or mechanically cut the captured material. FIG. 9F shows the outer catheter 220 advancing over the inner catheter 230 past the lesion material 212, while the laser cutting tool 225 ablates the lesion material 212 using laser light from the laser source 223, as the gripper 235 holds the lesion material 212. The ablation separates a portion of the lesion material 212 from the wall of the vessel 211.

In block S815, once the material has been separated, the inner catheter is retracted through the outer catheter while the material is captured by the capture tool in order to retrieve the material from the bodily lumen entirely. FIG. 9G shows the inner catheter 230 being retracted through the outer catheter 220 while the gripper 235 continues to hold onto the separated portion of the lesion material 212. This enables retrieval of the separated portion of the lesion material 212 from a proximal end of the outer catheter 220.

In block S816, it is determined whether the removal is complete. In other words, it is determined whether the ablation or mechanical cutting, and subsequent retrieval, of the portion of the material has reduced the size of the material by a sufficient, e.g., enabling substantially unrestricted blood flow or clearing of an object. The determination may be made by the user. For example, the ablation may be considered complete when the material can no longer be ablated since the only remaining portion is outside the diameter of the cutting tool. The determination of whether the removal is complete may be made using intravascular imaging, as discussed above with reference to FIGs. 6 and 7, and/or using any compatible external imager, such as an x-ray imaging device, an ultrasound imaging device, a CT scanner, or an MR imaging device, for example, as discussed above with reference to FIG. 1.

When it is determined that the removal is not complete (block S816: No), the process returns to block S812 to blocks S812 to S815 with respect to any remaining portion of the material. When it is determined that the removal is complete (block S81: Yes), the process continues on to block S817, according to which the outer catheter is withdrawn from the vessel. FIG. 9H shows the outer catheter 220 (and the inner catheter 230 to the extent it is still inserted within the outer catheter 220) being withdrawn from the vessel 211, and FIG. 9I shows the vessel 211 with the guide wire 115 still present following withdrawal of the outer catheter 220. Remnants of the lesion material 212 are shown in the vessel 211, which substantially open to unobstructed blood flow.

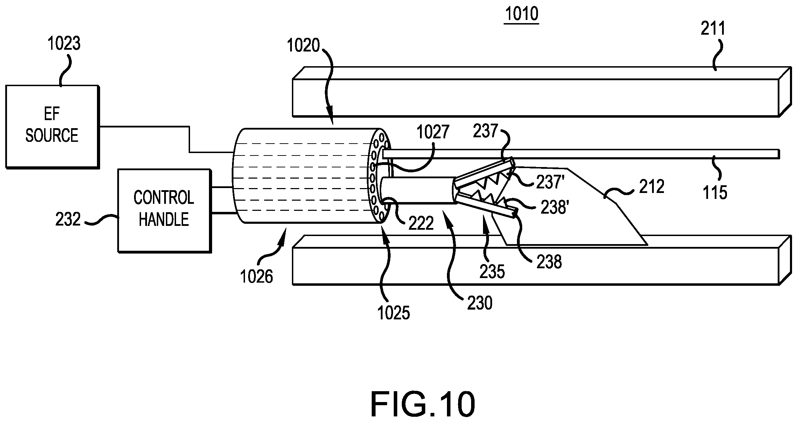

As previously mentioned, the embodiments discussed above with reference to a laser cutting tool and/or a laser source may likewise be applied to other types of treatment devices. In this context, FIGs. 10, 11 and 12 show basic versions of the other types of treatment catheters and cutting tools that may be configured to implement each of the embodiments discussed above.

FIG. 10 is a simplified plan view of a catheter system including an EM cutting tool and a gripper as the capture tool for removing lesion material from a vessel of a subject, according to a representative embodiment.

Referring to FIG. 10, catheter system 1010 is an EM catheter system shown positioned within the vessel 211 adjacent to the lesion material 212 during treatment for removing the lesion material 212. The catheter system 1010 includes the outer catheter 1020 and the inner catheter 230 configured for insertion through the vessel 211, e.g., along the optional guide wire 115. The outer catheter 1020 is substantially the same as the outer catheter 220, except for an EM wave cutting tool 1025 in place of the laser cutting tool 225. The inner catheter 230 passes through the catheter lumen 222 formed longitudinally in the outer catheter 220, and is moveable therein, as discussed above.

The EM wave cutting tool 1025 delivers RF or MW electric current generated by an EM wave source 1023. The EM wave cutting tool 1025 includes one or more signal lines 1026 (indicated by representative dashed lines) passing longitudinally through the outer catheter 1020 and corresponding electrodes 1027 at the distal end of the outer catheter 1020. The electrodes 1027 are arranged in electrode pairs configured to emit the EM electric current generated by the EM wave source 1023 from the distal end of the outer catheter 1020. That is, the EM electric current may be provided via one of more pairs of electrodes 1027 (bipolar configuration). In an alternative configuration, the EM electric current may be provided via one or more electrodes 1027 and a patch outside the subject's body or another catheter with electrodes inside the subject's body (monopolar configuration). The EM electric current ablates a portion of the lesion material 212 while the lesion material 212 is captured by the gripper 235, which is positioned at the distal end of the inner catheter 230.

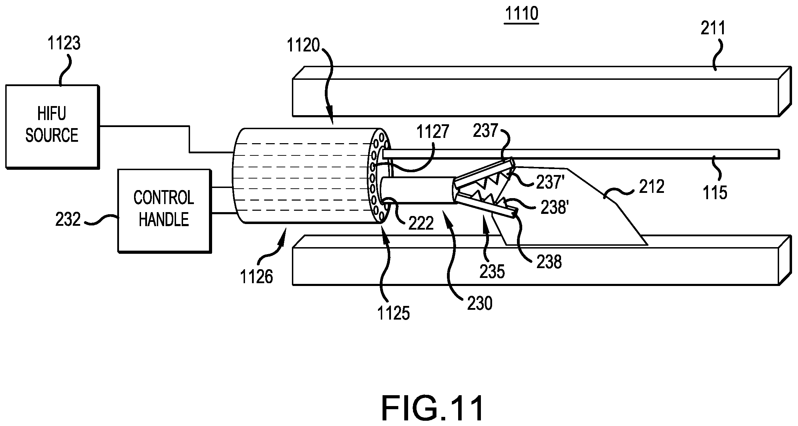

FIG. 11 is a simplified plan view of a catheter system including a HIFU cutting tool and a gripper as the capture tool for removing lesion material from a vessel of a subject, according to a representative embodiment.

Referring to FIG. 11, catheter system 1110 is a HIFU catheter system shown positioned within the vessel 211 adjacent to the lesion material 212 during treatment for removing the lesion material 212. The catheter system 1110 includes the outer catheter 1120 and the inner catheter 230 configured for insertion through the vessel 211, e.g., along the optional guide wire 115. The outer catheter 1120 is substantially the same as the outer catheter 220, except for a HIFU cutting tool 1125 in place of the laser cutting tool 225. The inner catheter 230 passes through the catheter lumen 222 formed longitudinally in the outer catheter 1120, and is moveable therein, as discussed above.

The HIFU cutting tool 1125 delivers ultrasonic signals generated in response to electric signal generated by a HIFU source 1123. The HIFU cutting tool 1125 includes signal lines 1126 (indicated by representative dashed lines), each of which includes two leads, passing longitudinally through the outer catheter 1120, and corresponding one or more ultrasound transducers 1127. The ultrasound transducers 1127 are configured to emit ultrasonic waves from the distal end of the outer catheter 1120 in response to the electrical signals. The ultrasonic waves may be in a frequency range from about 0.6 MHz to about 15 MHz. The ultrasonic waves ablate a portion of the lesion material 212 while the lesion material 212 is captured by the gripper 235, which is positioned at the distal end of the inner catheter 230.

In an embodiment, the ultrasound transducers 1127 may be configured to also provide forward looking IVUS imaging (including color and/or spectral Doppler imaging), as discussed above with reference to inner IVUS imager 665 and outer IVUS imager 765 in FIGs. 6 and 7. In this configuration, the ultrasound transducers 1127 can be used in an IVUS imaging mode when performing the forward looking IVUS imaging in order to navigate to the lesion material 212 and capture it, and then used in an HIFU mode for ablating the lesion material 212. The ultrasound transducers 1127 may also be used for IVUS imaging to assess progress and results of the ablation during (intermittently) and after the procedure, respectively.

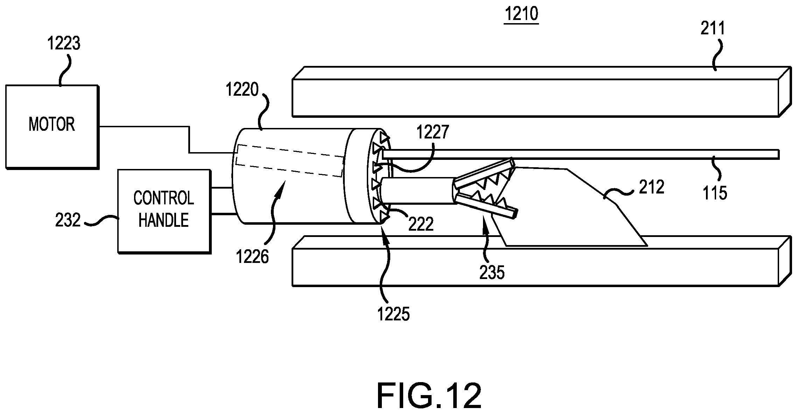

FIG. 12 is a simplified plan view of a catheter system including a mechanical cutting tool and a gripper as the capture tool for removing lesion material from a vessel of a subject, according to a representative embodiment.

Referring to FIG. 12, catheter system 1210 is a mechanical catheter system shown positioned within the vessel 211 adjacent to the lesion material 212 during treatment for removing the lesion material 212. The catheter system 1210 includes the outer catheter 1220 and the inner catheter 230 configured for insertion through the vessel 211, e.g., along the optional guide wire 115. The outer catheter 1220 is substantially the same as the outer catheter 220, except for a mechanical cutting tool 1225 in place of the laser cutting tool 225. The inner catheter 230 passes through the catheter lumen 222 formed longitudinally in the outer catheter 1120, and is moveable therein, as discussed above.

The mechanical cutting tool 1225 rotates under control of a motor 1223. The mechanical cutting tool 1225 includes a drive train 1226 passing through the outer catheter 1220 and a rotating or translating cutter 1227 with one or more cutting blades connected to the distal end of the drive train 1226. The rotating or translating cutter 1227 is configured cut a portion of the lesion material 212 with the one or more cutting blades while being rotated and/or translated by the motor 1223 and the drive train 1226. The cutter 1227 thus mechanically cuts a portion of the lesion material 212 while the lesion material 212 is captured by the gripper 235, which is positioned at the distal end of the inner catheter 230.

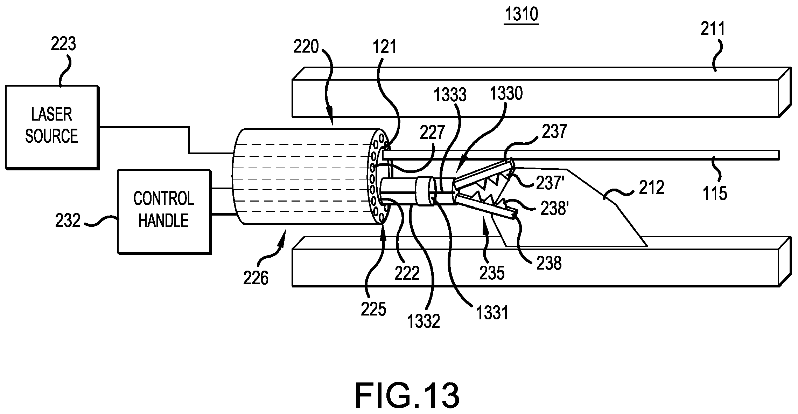

As mentioned above, at least one pull wire may be attached to a distal end of the inner catheter to enable steering of the inner catheter. FIG. 13 is a simplified plan view of a catheter system including a laser cutting tool and a gripper as the capture tool, and pull wires for manipulating the gripper, according to a representative embodiment.

Referring to FIG. 13, catheter system 1310 is a laser catheter system, such as the catheter system 200 discussed above with reference to FIG. 2. The catheter system 1310 is shown positioned within the vessel 211 adjacent to the lesion material 212 during treatment for removing the lesion material 212. The catheter system 1310 includes the outer catheter 220 and the inner catheter 1330 configured for insertion through the vessel 211, e.g., along the optional guide wire 115. The inner catheter 1330 is substantially the same as the inner catheter 230 with the addition of a pull wire mechanism for steering the inner catheter 1330 and actuating the gripper 235. In particular, the inner catheter 1330 includes a pull ring 1331 attached to the inner catheter 1330 near its distal end. A steering pull wire 1332 is attached to the bottom portion of the pull ring 1331, and is configured to enable steering of the inner catheter 1330. For example, pulling the steering pull wire 1332 would bend the inner catheter 1330 downward and rotating the inner catheter 1330 would redirect the distal end. Additional steering pull wires may be attached to other portions of the pull ring 1331 to similarly enable bending of the inner catheter 1330 in different directions. An actuation pull wire 1333 is attached to the proximal end of the gripper 235, and is configured to actuate (open and close) the opposable jaws 237 and 238. For example, pulling the actuation pull wire 1333 would close the opposable jaws 237 and 238 and releasing/pushing the actuation pull wire 1333 would open the opposable jaws 237 and 238. Other configuration of steering and/or actuation pull wires may be implemented without departing from the scope of the present teachings.

It is understood that, in alternative embodiments, the pull wire mechanism, comprising the pull ring 1331 and the at least one steering pull wire 1332, described with respect to the inner catheter 1330 may be provided on the outer catheter 220 instead of or in addition to the pull wire mechanism provided on the inner catheter 1330.

In accordance with various embodiments of the present disclosure, the methods described herein may be implemented using a hardware computer system that executes software programs stored on non-transitory storage mediums. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

Although catheter systems for treating lesions have been described with reference to exemplary embodiments, it is understood that the words that have been used are words of description and illustration, rather than words of limitation. Changes may be made within the purview of the appended claims, as presently stated and as amended, without departing from the scope and spirit of the embodiments. Although catheter systems for treating lesions have been described with reference to particular means, materials and embodiments, it is not intended to be limited to the particulars disclosed; rather catheter systems for treating lesions extends to all functionally equivalent structures, methods, and uses such as are within the scope of the appended claims.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The Abstract of the Disclosure is provided to comply with 37 C.F.R. §1.72(b) and is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the true spirit and scope of the present disclosure. Thus, to the maximum extent allowed by law, the scope of the present disclosure is to be determined by the broadest permissible interpretation of the following claims and their equivalents and shall not be restricted or limited by the foregoing detailed description.

### Additional embodiments

Embodiment 1. A catheter system for removing material from a bodily lumen of a subject, the catheter system comprising:
an outer catheter configured for insertion in the bodily lumen, wherein the outer catheter defines a first lumen and comprises a cutting tool at a distal tip of the outer catheter; and
an inner catheter configured for insertion in the bodily lumen through the first lumen of the outer catheter, wherein the inner catheter comprises a capture tool configured to extend from the distal tip of the outer catheter and to capture the material,
wherein the outer catheter is further configured to advance over the inner catheter toward the captured material and to remove the captured material from the bodily lumen while the material is captured by the capture tool.

Embodiment 2. The catheter system of embodiment 1, wherein the cutting tool is a laser cutting tool comprising a plurality of optical fibers passing through the outer catheter, wherein the plurality of optical fibers are configured to emit laser light, generated by a laser source, from the distal tip of the outer catheter for removing the material, while the material is captured by the capture tool.

Embodiment 3. The catheter system of embodiment 1, wherein the cutting tool is a mechanical cutting tool comprising a drive train passing through the outer catheter, wherein the drive train is configured to rotate or translate one or more cutting blades positioned on the distal tip of the outer catheter for physically cutting the material, while the material is captured by the capture tool.

Embodiment 4. The catheter system of embodiment 1, wherein the cutting tool is an electromagnetic (EM) wave cutting tool comprising at least one electrode configured to emit radio frequency (RF) or microwave frequency (MW) signals, generated by an EM source, from the distal tip of the outer catheter for removing the material, while the material is captured by the capture tool.

Embodiment 5. The catheter system of embodiment 1, wherein the cutting tool is a high-intensity focused ultrasound (HIFU) cutting tool comprising at least one ultrasound transducer configured to emit HIFU signals from the distal tip of the outer catheter for removing the material, while the material is captured by the capture tool.

Embodiment 6. The catheter system of embodiment 1, wherein the capture tool comprises a gripper comprising a pair of opposable jaws operable to clamp onto the material for capturing the material.

Embodiment 7. The catheter system of embodiment 1, wherein the capture tool comprises a snare including one or more loops configured to expand when the inner catheter extends from the distal tip of the outer catheter for capturing the material.

Embodiment 8. The catheter system of embodiment 1, wherein the capture tool comprises a helix configured to embed into the material by rotation for capturing the material.

Embodiment 9. The catheter system of embodiment 1, wherein the capture tool comprises an expandable mesh configured to expand when the inner catheter extends from the distal tip of the outer catheter in order to anchor into a portion of the material for capturing the material, wherein the expandable mesh is formed of a nitinol material having shape memory properties.

Embodiment 10. The catheter system of embodiment 1, wherein at least one of the outer catheter or the inner catheter further comprises an intravascular ultrasound (IVUS) imager configured to provide an ultrasound image of at least a portion of the material in real time.

Embodiment 11. The catheter system of embodiment 2, further comprising: an optical imager configured to provide an optical image of at least a portion of the material in real time, wherein the optical imager is configured to receive light reflected from the material via the optical fibers configured to emit the laser light or via other optical fibers passing through the outer catheter.

Embodiment 12. The catheter system of embodiment 1, wherein the bodily lumen comprises a vessel, and the material comprises lesion material from a lesion on a vessel wall of the vessel.

Embodiment 13. The catheter system of embodiment 1, wherein the bodily lumen comprises a portion of an organ in the subject, and the material comprises tissue to be removed from the organ.

Embodiment 14. The catheter system of embodiment 1, wherein the bodily lumen comprises a lumen in a heart of the subject, and the material comprises a portion of the heart to be removed from the heart.

Embodiment 15. A method of removing material in a bodily lumen of a subject using a catheter system comprising an outer catheter defining a first lumen and comprising a cutting tool at a distal tip of the outer catheter, and an inner catheter comprising a capture tool, the method comprising:
advancing the outer catheter through the bodily lumen to the material;
advancing the inner catheter through the first lumen of the outer catheter toward the material until the capture tool of the inner catheter extends from the distal tip of the outer catheter;
capturing the material using the capture tool;
   cutting and/or ablating the captured material in the bodily lumen using the cutting tool at the distal tip of the outer catheter; and
retracting the inner catheter through the first lumen of the outer catheter to retrieve the captured material from the bodily lumen while the material is captured by the capture tool.

## Claims

1. A catheter system for removing material from a bodily lumen of a subject, the catheter system comprising:
an outer catheter configured for insertion in the bodily lumen, wherein the outer catheter defines a first lumen and comprises a cutting tool at a distal tip of the outer catheter; and
an inner catheter configured for insertion in the bodily lumen through the first lumen of the outer catheter, wherein the inner catheter comprises a capture tool configured to extend from the distal tip of the outer catheter and to capture the material,
wherein the outer catheter is further configured to advance over the inner catheter toward the captured material and to remove the captured material from the bodily lumen while the material is captured by the capture tool.

2. The catheter system of claim 1, wherein the cutting tool is a laser cutting tool comprising a plurality of optical fibers passing through the outer catheter, wherein the plurality of optical fibers are configured to emit laser light, generated by a laser source, from the distal tip of the outer catheter for removing the material, while the material is captured by the capture tool.

3. The catheter system of claim 1, wherein the cutting tool is a mechanical cutting tool comprising a drive train passing through the outer catheter, wherein the drive train is configured to rotate or translate one or more cutting blades positioned on the distal tip of the outer catheter for physically cutting the material, while the material is captured by the capture tool.

4. The catheter system of claim 1, wherein the cutting tool is an electromagnetic (EM) wave cutting tool comprising at least one electrode configured to emit radio frequency (RF) or microwave frequency (MW) signals, generated by an EM source, from the distal tip of the outer catheter for removing the material, while the material is captured by the capture tool.

5. The catheter system of claim 1, wherein the cutting tool is a high-intensity focused ultrasound (HIFU) cutting tool comprising at least one ultrasound transducer configured to emit HIFU signals from the distal tip of the outer catheter for removing the material, while the material is captured by the capture tool.

6. The catheter system of any one of claims 1 to 5, wherein the capture tool comprises a gripper comprising a pair of opposable jaws operable to clamp onto the material for capturing the material.

7. The catheter system of any one of claims 1 to 5, wherein the capture tool comprises a snare including one or more loops configured to expand when the inner catheter extends from the distal tip of the outer catheter for capturing the material.

8. The catheter system of any one of claims 1 to 5, wherein the capture tool comprises a helix configured to embed into the material by rotation for capturing the material.

9. The catheter system of any one of claims 1 to 5, wherein the capture tool comprises an expandable mesh configured to expand when the inner catheter extends from the distal tip of the outer catheter in order to anchor into a portion of the material for capturing the material, wherein the expandable mesh is formed of a nitinol material having shape memory properties.

10. The catheter system of any one of claims 1 to 9, wherein at least one of the outer catheter or the inner catheter further comprises an intravascular ultrasound (IVUS) imager configured to provide an ultrasound image of at least a portion of the material in real time.

11. The catheter system of any one of claims 1 to 9, further comprising:
an optical imager configured to provide an optical image of at least a portion of the material in real time, wherein the optical imager is configured to receive light reflected from the material via the optical fibers configured to emit the laser light or via other optical fibers passing through the outer catheter.

12. The catheter system of any one of claims 1 to 11, wherein the bodily lumen comprises a vessel, and the material comprises lesion material from a lesion on a vessel wall of the vessel.

13. The catheter system of any one of claims 1 to 12, wherein the bodily lumen comprises a portion of an organ in the subject, and the material comprises tissue to be removed from the organ.

14. The catheter system of any one of claims 1 to 13, wherein the bodily lumen comprises a lumen in a heart of the subject, and the material comprises a portion of the heart to be removed from the heart.

15. A method of removing material in a bodily lumen of a subject using a catheter system comprising an outer catheter defining a first lumen and comprising a cutting tool at a distal tip of the outer catheter, and an inner catheter comprising a capture tool, the method comprising:
advancing the outer catheter through the bodily lumen to the material;
advancing the inner catheter through the first lumen of the outer catheter toward the material until the capture tool of the inner catheter extends from the distal tip of the outer catheter;
capturing the material using the capture tool;
cutting and/or ablating the captured material in the bodily lumen using the cutting tool at the distal tip of the outer catheter; and
retracting the inner catheter through the first lumen of the outer catheter to retrieve the captured material from the bodily lumen while the material is captured by the capture tool.
